Europäisches Patentamt

European Patent Office

Office européen des brevets

(11) Publication number: **0 447 100 A1**

(12) # EUROPEAN PATENT APPLICATION

(21) Application number: 91301806.5

(22) Date of filing: 05.03.91

(51) Int. Cl.[5]: **A61K 9/22, A61K 9/26, A61K 47/42, A61K 47/48**

(30) Priority: 06.03.90 GB 9004950

(43) Date of publication of application:
18.09.91 Bulletin 91/38

(84) Designated Contracting States:
CH DE FR GB IT LI NL

(71) Applicant: KELCO INTERNATIONAL LIMITED
Westminster Tower 3 Albert Embankment
London SE1 7RZ (GB)

(72) Inventor: Barker, Sidney Alan
1 Abdon Avenue, Selly Oak,
Birmingham, B29 4NT (GB)
Inventor: Gray, Charles John
66 School Road
Hall Green, Birmingham B28 8JA (GB)
Inventor: Hofmann, Martin
Corner Cottage, The Birches
Birches Drive, Stroud GL5 1SS (GB)

(74) Representative: Thompson, John Dr. et al
Merck & Co., Inc. European Patent
Department Terlings Park Eastwick Road
Harlow, Essex CM20 2QR (GB)

(54) Controlled release formulation.

(57) A controlled release formulation based on a gel matrix for controlled release of a pharmaceutical, a foodstuff, or as a component of a diagnostic assay apparatus.

EP 0 447 100 A1

Jouve, 18, rue Saint-Denis, 75001 PARIS

Fig. 3.

# CONTROLLED RELEASE FORMULATION

The present invention relates to a controlled release formulation. More particularly, the invention relates to a formulation based on a gel matrix, and to various applications of this formulation, for example as a controlled release pharmaceutical composition, as a composition for the controlled release of a foodstuff additive, or as one component of a diagnostic assay apparatus.

Various devices whereby a desired ingredient is released in a controlled manner from a gel matrix are known from the art. For example, GB-A-2207353 describes a calcium-free formulation comprising up to 45% by weight of a pH-dependent alginic acid salt, e.g. sodium alginate, and up to 35% by weight of a pH-dependent hydrocolloid gelling agent, e.g. hydroxypropylmethyl cellulose, for the controlled release of a basic pharmaceutical agent, e.g. verapamil. A delivery system is described in EP-A-0202819 consisting of an active ingredient entrapped in a cross-linked alginate or carrageenenate matrix; release of active ingredient from the matrix is stated to be prolonged and controlled.

Formulations based solely on the entrapment of an active ingredient in a cross-linked gel matrix possess various drawbacks. For example, molecules whose diameter is smaller than that of the matrix pores, and particularly water-soluble molecules, have a distinct tendency to "leach" from the formulation. This renders difficult the preparation of formulations from which the release of active ingredient can be effected in a controlled manner. Moreover, in the case of expensive ingredients, such as many drugs, premature "leaching" of the active ingredient from the formulation has significant economic disadvantages.

We have now found that these drawbacks can be overcome by providing a formulation in which the ingredient is bound to a protein which is itself entrapped in a gel matrix. The protein-bound ingredient remains associated with the matrix until such a time as the formulation is brought into contact with a proteolytic enzyme for which the entrapped protein is an appropriate substrate, whereupon the protein is degraded and the ingredient thereby released.

Accordingly, in one aspect the present invention provides a controlled release formulation comprising:

a gel matrix;

a protein entrapped in the gel matrix; and

an ingredient capable of binding to the entrapped protein;

whereby, upon exposure of the formulation to a proteolytic enzyme-containing environment, the protein is degraded and the ingredient released.

By "ingredient" is meant any substance the release of which from the formulation is desirably effected in a controlled manner. Examples of suitable ingredients include pesticides, herbicides, germicides, biocides, algicides, rodenticides, fungicides, insecticides, antioxidants, plant growth promoters, plant growth inhibitors, preservatives, disinfectants, sterilization agents, catalysts, chemical reactants, fermentation agents, foods, food supplements, foodstuff additives, nutrients, cosmetics, drugs, vitamins, sex sterilants, fertility inhibitors, fertility promoters, air purifiers, microorganism attenuators, and indicator substances such as food dyes.

It will be appreciated that the entrapped protein will be an appropriate substrate for the proteolytic enzyme, otherwise release of the ingredient from the formulation will not occur at the desired location. The protein must also, of course, be capable of being entrapped in the gel matrix. Further to these requirements, however, no additional restriction on the nature of the protein and of the proteolytic enzyme is envisaged.

The proteolytic enzyme itself may be present in situ in a living human or animal body, or may be used in isolated and/or purified form in vitro. The controlled release formulation according to the invention is accordingly amenable to a range of applications.

Thus, in a further aspect the present invention provides a controlled release pharmaceutical composition comprising:

a gel matrix;

a protein entrapped in the gel matrix;

a drug capable of binding to the entrapped protein; and

optionally pharmaceutically acceptable excipients;

whereby, upon exposure of the composition to a proteolytic enzyme-containing environment, the protein is degraded and the drug released.

As used herein, the term "drug" includes any physiologically or pharmacologically active substance that produces any localized or systemic effect or effects in animals, including mammals, humans and primates. Particular animals thus include domestic household, sport or farm animals such as sheep, goats, cattle, horses and pigs; and laboratory animals such as mice, rats and guinea pigs; as well as fishes, birds, reptiles and other zoo animals.

It will be appreciated that the sole requirement for the drug used will be its ability to bind to the entrapped protein. In principle, therefore, any known drug can be employed as long as an acceptable protein can be found to which it will bind. By "acceptable protein" is meant any protein capable of being entrapped in the gel matrix.

Examples of beneficial drugs are disclosed in Remington's Pharmaceutical Sciences, 17th Ed., 1985, published by Mack Publishing Co., Easton, Pennsylvania, USA; and in Goodman and Gilman, The Pharmacological Basis of Therapeutics, 7th Ed., 1985, published by Macmillan, London.

The active drugs that can be employed include inorganic and organic compounds that act on the peripheral nerves, adrenergic receptors, cholinergic receptors, nervous system, skeletal muscles, cardiovascular system, smooth muscles, blood circulatory system, synaptic sites, neuroeffector junctional sites, endocrine and hormone systems, immunological system, reproductive system, skeletal system, autocoid systems, and alimentary and excretory systems, as well as inhibitors of the autocoid and histamine systems. Examples of suitable drugs include materials that act on the central nervous system such as hypnotics and sedatives, including pentobarbital sodium, phenobarbital, secobarbital, thiopental and mixtures thereof; heterocyclic hypnotics and sedatives such as dioxopiperidines and glutarimides; hypnotics and sedatives such as amides and ureas, exemplified by diethylisovaleramide and α-bromoisovaleryl urea; hypnotic and sedative urethanes and disulphanes; psychic energizers such as isocoboxazid, nialamide, phenelzine, imipramine, amitryptyline hydrochloride, tranylcypromine, pargylene, and protryptyline hydrochloride; tranquilizers such as chloropromazine, promazine, fluphenazine, reserpine, deserpidine, meprobamate, and benzodiazepines such as chlordiazepoxide; anticonvulsants such as primidone, enitabas, diphenylhydantoin, ethyltion, pheneturide and ethosuximide; muscle relaxants and antiparkinsonian agents such as mephenesin, methocarbamol, cyclobenzaprine, trihexylphenidyl, levodopa/carbidopa, and biperiden; antihypertensives such as α-methyldopa, L-β-3,4-dihydroxyphenylalanine, and pivaloyloxyethyl ester of α-methyldopa hydrochloride dihydrate; analgesics such as morphine, codeine, meperidine and nalorphine; antipyretics and anti-inflammatory agents such as aspirin, indomethacin, sodium indomethacin trihydrate, salicylamide, naproxen, colchicine, fenoprofen, sulindac, diflunisal, diclofenac, indoprofen and sodium salicylamide; local anaesthetics such as procaine, lidocaine, maepaine, piperocaine, tetracaine and dibucaine; antispasmodics and muscle contractants such as atropine, scopolamine, methscopolamine, oxyphenonium and papaverine; prostaglandins such as $PGE_1$, $PGE_2$, $PGF_{1\alpha}$, $PGF_{2\alpha}$ and PGA; antimicrobials and antiparasitic agents such as penicillin, tetracycline, oxytetracycline, chlorotetracycline, chloramphenicol, thiabendazole, ivermectin, and sulphonamides; antimalarials such as 4-aminoquinolines, 8-aminoquinolines and pyrimethamine; hormonal agents such as dexamethasone, prednisolone, cortisone, cortisol and triamcinolone; androgenic steroids such as methyltestosterone and fluoxymesterone; oestrogenic steroids such as 17β-oestradiol, α-oestradiol, oestriol, α-oestradiol 3-benzoate, and 17-ethynyl oestradiol-3-methyl ether; progestational steroids such as progesterone, 19-nor-pregn-4-ene-3,20-dione, 17-hydroxy-19-nor-17α-pregn-5(10)-ene-20-yn-3-one, 17α-ethynyl-17-hydroxy-5(10)-oestren-3-one, and 9β,10α-pregna-4,6-diene-3,20-dione; sympathomimetic drugs such as adrenaline, phenylpropanolamine hydrochloride, amphetamine, ephedrine and noradrenaline; hypotensive drugs such as hydralazine; cardiovascular drugs such as procainamide, procainamide hydrochloride, amyl nitrite, nitroglycerin, dipyridamole, sodium nitrate and mannitol nitrate; diuretics such as chlorothiazide, acetazolamide, methazolamide, hydrochlorothiazide, amiloride hydrochloride, flumethiazide, ethacrynic acid and furosemide; antiparasitics such as bephenium, hydroxynaphthoate, dichlorophen and dapsone; neoplastics such as mechlorethamine, uracil mustard, 5-fluorouracil, 6-thioguanine and procarbazine; β-blockers such as pindolol, propranolol, practolol, metoprolol, oxprenolol, timolol, timolol maleate, atenolol, alprenolol, and acebutolol; hypoglycaemic drugs such as insulin, isophane insulin, protamine zinc insulin suspension, globin zinc insulin, extended insulin zinc suspension, tolbutamide, acetohexamide, tolazamide and chlorpropamide; antiulcer drugs such as cimetidine and ranitidine; nutritional agents such as ascorbic acid, niacin, nicotinamide, folic acid, choline, biotin, pantothenic acid, and vitamin $B_{12}$; essential amino acids; essential fats; eye drugs such as timolol, timolol maleate, pilocarpine, pilocarpine nitrate, pilocarpine hydrochloride, dichlorphenamide, atropine, atropine sulphate, scopolamine and eserine salicylate; electrolytes such as calcium gluconate, calcium lactate, potassium chloride, potassium sulphate, sodium chloride, potassium fluoride, sodium fluoride, ferrous lactate, ferrous gluconate, ferrous sulphate, ferrous fumarate and sodium lactate; drugs that act on α-adrenergic receptors such as clonidine hydrochloride; and quinoline and naphthyridine carboxylic acids.

A preferred class of drug comprises the tetracycline series, especially chlorotetracycline, demeclocycline and tetracycline itself.

The drug can be in various forms, such as uncharged molecules, molecular complexes, and pharmacologically acceptable salts such as hydrochloride, hydrobromide, sulphate, laurylate, palmitate, phosphate, nitrite, borate, acetate, maleate, tartrate, oleate and salicylate salts. For acid drugs, salts of metals, amines or organic cations, for example quaternary ammonium salts, can be used. Derivatives of drugs such as esters, ethers and amides which have suitable solubility characteristics can be used alone or mixed with other drugs. Moreover, a water-insoluble drug can be used in a water-soluble derivative form thereof and, on its release from the compo-

EP 0 447 100 A1

sition, can, for example, be converted by enzymes, or hydrolyzed by body pH or other metabolic processes, to the original form, or to a biologically active form thereof.

The amount of drug to be incorporated into the composition will depend upon such factors as the nature of the drug concerned, and the severity of the disorder to be treated. In general, amounts of 0.05 to 60% by weight of the final composition are suitable, preferably 5.0 to 50%.

The optional pharmaceutically acceptable excipients assist in the manufacture of the composition and include conventional materials such as lactose, magnesium stearate, talc and sorbitol.

A notable advantage of the pharmaceutical composition according to the invention is site-specificity. Thus, by appropriate choice of protein embedded in the gel matrix, the composition can be formulated so as to remain intact upon passage through the body until it encounters a particular enzyme for which the protein is the substrate. Only at this point will the protein be degraded and the drug released. The drug can hence be targetted accurately at its intended site of activity, thereby minimising any adverse side-effects due to interaction of the drug with other sites throughout the body.

In order to ensure that release of drug into the stomach cavity is minimised, the composition can be provided with a conventional enteric layer. A variety of materials can be used for such enteric layers or coatings, such materials including a number of polymeric acids and mixtures of polymeric acids with such materials as shellac, cetyl alcohol and cellulose acetate.

The present invention is not confined to orally administrable pharmaceutical compositions, however, and equally includes within its scope pharmaceutical compositions for topical, rectal and parenteral administration. The compositions are preferably in unit dosage forms such as tablets, pills, capsules, suppositories, powders, granules, or sterile parenteral solutions or suspensions, and may conveniently be formulated by methods known from the art, for example as described in Remington's Pharmaceutical Sciences, 17th Ed., 1985.

In a further aspect the present invention provides a composition for the controlled release of a foodstuff additive comprising:

a gel matrix;

a protein entrapped in the gel matrix; and

a foodstuff additive capable of binding to the entrapped protein;

whereby, upon exposure of the composition to a proteolytic enzyme-containing environment, the protein is degraded and the foodstuff additive released.

The expression "foodstuff additive" is used herein in its broadest sense. In particular, this expression encompasses such things as flavouring agents, perfumes, colouring agents and sweetening agents, and mixtures thereof. As before, the only requirement is that the foodstuff additive will be capable of binding to the entrapped protein.

The foodstuff additive-containing composition according to the invention may be used in a variety of products or vehicles including chewing gum and confectionery products, food products, tobacco, and proprietary products such as toothpaste and denture adhesive.

The composition is particularly well suited to products requiring the gradual release of flavour over an extended period of time. It can thus be employed particularly advantageously to impart a sustained flavour to products such as chewing gum, which is well known to lose its flavour sensation rapidly after only a short initial chewing period.

Other situations in which the above composition may find application include the food processing industry, where it may be desired to release a given ingredient sustainedly over the duration of the processing cycle; and situations in which a particular flavouring ingredient is unstable and liable to decomposition. For example, a dry-mix dessert may contain an unstable flavouring ingredient; in this case, it is clearly desirable that the ingredient concerned should be released intact upon addition of milk, and not decomposed prior to usage of the product.

Examples of flavouring agents useful as the foodstuff additive include synthetic flavour oils, fruit essences and natural flavour oils derived from such sources as plants, leaves and flowers; as well as mixtures of the foregoing. Particular examples include spearmint oil, peppermint oil, cinnamon oil and oil of wintergreen (methyl salicylate); citrus oils derived from sources such as lemon, orange, grape, lime and grapefruit; fruit essences derived from sources such as apple, strawberry, cherry and pineapple; and extracts such as kola extract.

The amount of flavouring agent employed is normally a matter of preference subject to such factors as flavour type, base type and strength desired. In general, amounts of 0.05 to 3.0% by weight of the final composition are suitable, with amounts of 0.3 to 1.5% being preferred and 0.7 to 1.2% being particularly preferred.

Useful colouring agents and fragrances may be selected from any of those found suitable for food, drug and cosmetic applications, especially those described in Kirk-Othmer's Encyclopaedia of Chemical Technology, Vol. 5, pages 857-884. They may be incorporated in amounts of up to 1% by weight, and preferably up to 0.6% by weight.

5

Suitable sweetening agents include water-soluble natural sweeteners such as monosaccharides, disaccharides and polysaccharides, for example xylose, ribose, glucose, mannose, galactose, fructose, dextrose, sucrose, invert sugar, maltose, partially hydrolyzed starch, or corn syrup solids and sugar alcohols such as sorbitol, xylitol, mannitol, dihydrochalcone, glycyrrhizin and stevia rebaudiana (stevioside); water-soluble artificial sweeteners such as the soluble saccharin salts, i.e. sodium or calcium saccharin salts, cyclamate salts, the free acid form of saccharin, and the synthetic sweetener 3,4-dihydro-6-methyl-1,2,3-oxathiazin-4-one 2,2-dioxide, particularly the potassium (acesulfame-K), sodium and calcium salts thereof; dipeptide-based sweeteners such as L-aspartyl-L-phenylalanine methyl ester; and mixtures thereof.

In general, the amount of sweetener employed will vary with the desired sweetness of a particular composition. This amount will normally be from 0.01 to 90% by weight when using an easily extractable sweetener. The water-soluble natural sweeteners are preferably used in amounts of from 25 to 75% by weight, and especially from 50 to 65% by weight of the final composition. In contrast, the water-soluble artificial sweeteners and the dipeptide-based sweeteners are preferably used in amounts of from 0.01 to 5.0% and especially from 0.05 to 0.5% by weight of the final composition. These amounts are necessary to achieve a desired level of sweetness independent from the flavour level achieved from the flavour oil.

By appropriate choice of the constituents of the controlled release formulation according to the invention, it has been found that release of the ingredient into the proteolytic enzyme-containing environment can be made linear with respect to time, and also with respect to concentration of proteolytic enzyme. In this way, release of the ingredient from a typical composition can be calibrated. Thus, a further application of the formulation according to the invention is as one component of a diagnostic assay apparatus. In another aspect, therefore, the present invention provides a component for a diagnostic assay apparatus comprising:

a gel matrix;

a protein entrapped in the gel matrix; and

an indicator substance capable of binding to the entrapped protein;

whereby, upon exposure of the said component to a proteolytic enzyme, the protein is degraded and the indicator substance released.

In use, the above-defined component will be placed in a suitable medium such as a liquid, e.g. water, in a suitable container. The sample, containing an amount of proteolytic enzyme to be determined by the assay, will be already present in the supernatant liquid, or will be introduced subsequently. The entrapped protein within the component will be so chosen as to give linear release of the indicator substance depending on the concentration of proteolytic enzyme in the assay sample. Upon degradation of the entrapped substrate protein by the proteolytic enzyme, the indicator substance will be released into the supernatant liquid at a rate determined by the concentration of proteolytic enzyme. Release of the indicator substance into the supernatant liquid can then be monitored by appropriate sensor means known from the art, thereby giving an accurate indication of the concentration of proteolytic enzyme in the original assay sample.

Suitable indicator substances for use in the diagnostic assay component according to the invention include chromogenic, fluorogenic and luminescent molecules. Release of these substances into the supernatant liquid results in a build-up of colour, fluorescence or luminescence which can be detected and monitored by appropriate sensor means, such as conventional chemiluminescence apparatus. Particular indicator substances include food dyes such as erythrosine, Evans Blue Vital (EBV), methyl green, rose bengal, acridine orange, tropaeolin 00, methyl orange, acid orange 12, amido black 10B, carminic acid sodium salt, carmosine, tartrazine and methylene blue, especially erythrosine and EBV.

The amount of indicator substance present in the assay component will generally depend upon its intensity as observed by the detection means employed. In general, the amounts used will suitably vary between 0.001 and 0.2%, and preferably between 0.01 and 0.04%, by weight of the finished component.

The above-described assay component has been found to function particularly well, i.e. to give the optimum linear relationship, when the proteolytic enzyme to be assayed is an endoprotease such as trypsin, chymotrypsin, kallikrein, high alkaline protease (HAP; especially HAP-PB92) and elastase. When the proteolytic enzyme to be assayed is an exoprotease, e.g. carboxypeptidase or aminopeptidase, the assay component will nonetheless be effective, but will be subject to an initial time-lag. This is because the entrapped protein will be degraded only one amino acid at a time, so the indicator substance will not be released into the medium until such a time as the particular amino acid to which the indicator substance is bound has been reached.

The gel matrix present in the controlled release formulation according to the invention is suitably a matrix of a gel-forming polysaccharide. Examples of suitable gel-forming polysaccharides include carboxymethylcellulose, carrageenin, guar gum, gum arabic, gum acacia and polyuronates. Particular polyuronates include alginates, pectates and xanthan gum, preferably alginates.

Alginates, which are derived from seaweed, are long linear copolymers of D-mannuronic acid and L-guluronic acid having molecular weights ranging from 4,000 to about 18,000. In order to manifest their gel-forming properties, the alginates must be in the form of salts with multivalent cations. Examples of suitable multivalent cations include calcium, magnesium, zinc, copper, manganese, aluminium and iron, and mixtures thereof. Preferred multivalent cations are calcium and zinc, and mixtures thereof. Monovalent salts of alginates are water-soluble, and hence of no direct utility in the formation of gels. However, the monovalent cations are readily displaced by multivalent cations, so monovalent alginate salts may therefore serve as useful precursors to the gel-forming multivalent alginate salts.

An additional advantage to be derived from using a multivalent alginate salt as the gel-forming agent is that the multivalent cation employed may assist in binding the active ingredient. For example, tetracyclines are well known to bind to divalent metal ions, in particular to $Ca^{2+}$ ions; this binding, in conjunction with binding of the tetracycline molecule to the entrapped protein, will advantageously contribute to the overall integrity of the gel matrix.

Examples of suitable proteins for use in the controlled release formulation according to the invention include casein and albumin. Each of these proteins is capable of binding to a wide variety of ingredients, and both are cleavable by endoproteases, affording release of the active ingredient in a controlled manner. The use of albumin is particularly advantageous, since this protein is known to form complexes with many drugs. Where albumin is employed as the entrapped protein and the gel-forming agent is a multivalent alginate salt, the multivalent cation in this case will advantageously be $Zn^{2+}$. Alternatively, where casein is used as the entrapped protein and the gel-forming agent is a multivalent alginate salt, the cation employed will advantageously be $Ca^{2+}$ or $Zn^{2+}$, or a mixture thereof.

The outward appearance of the controlled release formulation according to the invention will in general be determined by the application to which the particular formulation concerned will be put. Thus, the formulation may be presented in any suitable form conventionally employed in the relevant art. Representative forms include beads, pellets, films, fibres and tubes. Standard techniques for the preparation of such forms may be employed, as described, for example, in Remington's Pharmaceutical Sciences, 17th Ed., 1985. Moreover, if the formulation is presented in the form of beads or pellets, a plurality of these may advantageously be contained in a hard gelatin capsule prepared by methods known from the art. Typical beads or pellets measure from 0.5 to 4 mm in diameter; this is particularly advantageous for pharmaceutical use, since compositions formed from particles larger than 4 mm in diameter have a tendency to be retained in the stomach, thereby potentially giving rise to undesirable localised side-effects.

The invention further provides a method for the preparation of a controlled release formulation as defined above which comprises mixing a protein and an ingredient capable of binding thereto, whose release from the formulation is desirably effected in a controlled manner, with a gel-forming agent, the latter forming a gel matrix which entraps the protein together with the ingredient bound thereto.

The proportions of the ingredients utilised in the above-described process will reflect those desired in the final product. Suitable amounts of active ingredient for incorporation into the finished formulation have already been indicated above. The proportions of gel-forming agent to protein advantageously employed are from 0.5:1 to 10:1 by weight, preferably 1:1 by weight.

Where the gel-forming agent employed is a multivalent alginate salt, the gel matrix may conveniently be formed by adding an aqueous solution of the corresponding monovalent alginate salt, e.g. the sodium, potassium or lithium salt, dropwise into an aqueous solution of a multivalent cation, preferably a divalent cation such as calcium chloride or zinc chloride. The protein and the active ingredient can be added together with or separately from the monovalent alginate salt solution, or may be already present in the multivalent cation solution. Once the droplets of monovalent alginate salt solution contact the multivalent cation solution a gel matrix rapidly forms, which entraps the protein together with the ingredient bound thereto. The advantage of this approach is that mixing of the final product is not necessary, since the size of the droplets can be controlled within the preferred range to ensure sufficient intimate contact and ion exchange.

The monovalent alginate salt and the multivalent cation may be mixed in stoichiometric amounts, but the multivalent cation is preferably present in excess to ensure substantially complete replacement of the monovalent cation by the multivalent cation in the gel matrix structure. Thus, suitable concentrations of the aqueous monovalent alginate salt solution may be from 0.25 to 2.0% by weight, preferably 1.0% by weight; whilst suitable concentrations of the aqueous solution of multivalent cation may be from 0.5 to 4.0% by weight, preferably 1.0 to 2.0% by weight. The mixing of the components is best carried out at a temperature which is lower than that at which the protein-bound ingredient will volatilise. The addition is conveniently carried out at temperatures between 1°C and 25°C, and preferably at room temperature.

Once the gel matrix has been formed, the precipitate can then be filtered off, washed and dried. Drying of the gel matrix may be carried out by conventional methods such as air drying at room temperature or freeze drying, or combinations of these techniques. In general, drying is best effected at temperatures of 25°C or less; and drying at temperatures above the volatilisation point of the protein-bound ingredient is plainly not recommended. Once dried, the resulting formulation can then be formed into suitable sized and shaped particles as described above.

Particular embodiments of the invention will now be described by way of example and with reference to the accompanying drawings in which:

**Figure 1** depicts the release of dye from beads prepared from calcium alginate, casein and erythrosine, showing the absorbance of the supernatant after preparation, after addition of α-amylase and glucoamylase, and then after addition of trypsin;

**Figure 2** depicts the release of dye from calcium alginate-casein-EBV beads by the action of trypsin, showing the variation of absorbance of the supernatant with time, after addition of trypsin;

**Figure 3** depicts the release of dye from calcium alginate-casein-EBV beads by the action of trypsin, showing the variation of rate of release of dye with concentration of trypsin used;

**Figure 4** depicts the release of dye from calcium alginate-casein-EBV beads by the action of chymotrypsin, showing the variation of rate of release of dye with chymotrypsin concentration;

**Figure 5** depicts the release of dye from calcium alginate-casein-EBV beads by the action of kallikrein, showing the variation of rate of dye release with kallikrein concentration;

**Figure 6** depicts the release of dye from calcium alginate-casein-EBV beads by the action of HAP-PB92, showing the variation of rate of dye release with proteinase concentration;

**Figure 7** depicts the release of dye from calcium alginate-casein-EBV by the action of elastase showing the variation of rate of dye release with elastase concentration; and

**Figure 8** depicts the release of tetracyclin from tetracyclin-casein-calcium alginate beads, by plotting the absorbance of supernatant at 370 nm against time.

## Preparative Example A

### Retention of Proteins in Alginate Gel Beads

A solution of casein (5.0 g) in aqueous sodium hydroxide solution (150 cm$^3$, 0.033 M) was boiled for 10 min and then cooled, adjusted to pH 6.0 with 2 M hydrochloric acid, diluted to 250 cm$^3$ with water and then readjusted to pH 6.0 with 0.05 M hydrochloric acid. A mixture of equal volumes (2.5 cm$^3$ each) of this solution and sodium alginate solution (2.0% w/v) was extruded dropwise through a syringe needle, dropping from a height of 10.0 cm into a solution of 2% (w/v) calcium chloride solution (50 ml). Beads were formed and the absorbance of the supernatant at 280 nm was read immediately and then at intervals for 20 hr.

This experiment was repeated using zinc chloride solution, and also using bovine serum albumin (BSA) in place of casein. The results are shown in Table 1 below.

## Table 1

### Retention of Bovine Serum Albumin, and of Casein, in Zinc Alginate and Calcium Alginate Beads

| | Protein Retained in Beads[a] | | | |
|---|---|---|---|---|
| | Alginate/Albumin | | Alginate/Casein | |
| | Zinc Alginate[b] | Calcium Alginate[c] | Zinc Alginate[b] | Calcium Alginate[c] |
| Immediately after preparation | 93 | 56 | 100 | 99 |
| 1 hr after preparation | 91 | 24 | 99 | 99 |
| 2 hr after preparation | 91 | 8 | 98 | 99 |
| 20 hr after preparation | 91 | 8 | 93 | 90 |

[a] % of the protein incorporated in the beads which did not appear in the supernatant after the times shown.

[b] The pH of the zinc chloride solution used for the preparation of the beads was 5.5.

[c] The pH of the calcium chloride solution used for the preparation of the beads was 6.0.

From Table 1, it can be seen that casein incorporated into calcium alginate beads is retained over a long period of time. Absorbance measurements at 280 nm on the supernatant above calcium alginate-casein beads showed virtually no leakage of peptide or protein over the first two hours. By contrast more than 90% of albumin incorporated into beads had diffused out in 2 hours. On the other hand, both casein and albumin are effectively retained within zinc alginate beads.

Example 1

Incorporation of Dyes into Alginate and Alginate/Protein Gel Beads

A solution containing 1% sodium alginate, 1% casein and 200 μg/cm³ of erythrosine was dropped into 2% calcium chloride solution, as described in Preparative Example A. After 1 hr, the absorbance at 525 nm was read and compared with the absorbance of a solution obtained by dropping 200 μg/cm³ of erythrosine alone into the calcium chloride solution, this latter giving a reference corresponding nominally to zero dye entrapped.

The experiment was repeated with casein absent, and also with EBV, methyl orange, rose bengal or tartrazine in place of erythrosine, with and without casein, and the absorbances of the supernatants read at the appropriate wavelengths. The results are shown in Table 2(a) below.

This series was also repeated using zinc chloride in place of calcium chloride and albumin in place of casein. The results obtained are shown in Table 2(b) below.

## Table 2

## Release of Dyes from Alginate and Alginate/Protein Beads

### (a) Calcium Alginate and Calcium Alginate/Casein Beads

| Dye ($\lambda_{max}$ nm) | Absorbance standard[a] | Absorbance of supernatant due to released dye 1 hr after preparation (% of standard) | |
| --- | --- | --- | --- |
| | | Calcium Alginate Beads | Calcium Alginate/Casein Beads |
| Erythrosine (525) | 1.68 | 1.34 (80) | 0.11 ( 7) |
| EBV (600) | 0.465 | 0.33 (71) | 0.08 (17) |
| Methyl Orange (465) | 0.48 | 0.39 (81) | 0.40 (83) |
| Rose Bengal (540) | 0.73 | 0.60 (90) | 0.185(25) |
| Tartrazine (425) | 0.97 | 0.955(98) | 0.955(98) |

[a] Absorbance of the dye in the corresponding volume of solution with no alginate beads present.

## (b) Zinc Alginate and Zinc Alginate/Albumin Beads

| Dye ($\lambda_{max}$ nm) Absorbance standard[a] | | Absorbance of supernatant due to released dye 1 hr after preparation (% of standard). | |
|---|---|---|---|
| | | Zinc Alginate Beads | Zinc Alginate/ Albumin Beads |
| Erythrosine (525) | 1.70 | 1.48 (87) | 0.085( 5) |
| EBV (600) | 0.69 | 0.24 (35) | 0.055( 8) |
| Methyl Orange (465) | 0.49 | 0.44 (90) | 0.16 (33) |
| Rose Bengal (540) | 0.61 | 0.60 (98) | 0.105(17) |
| Tartrazine (425) | 0.955 | 0.91 (95) | 0.88 (92) |

[a] Absorbance of the dye in the corresponding volume of solution with no alginate beads present.

From Tables 2(a) and 2(b), it can be seen that erythrosine, EBV and rose bengal exhibit substantial binding in both calcium alginate-casein-dye beads and zinc alginate-albumin-dye beads. Moreover, methyl orange displays significant binding in zinc alginate-albumin-dye beads. Of the five dyes tested, only tartrazine was completely released from both calcium alginate-casein-dye beads and zinc alginate-albumin-dye beads.

Example 2

Release of Casein-Bound Erythrosine from Alginate Gel Beads

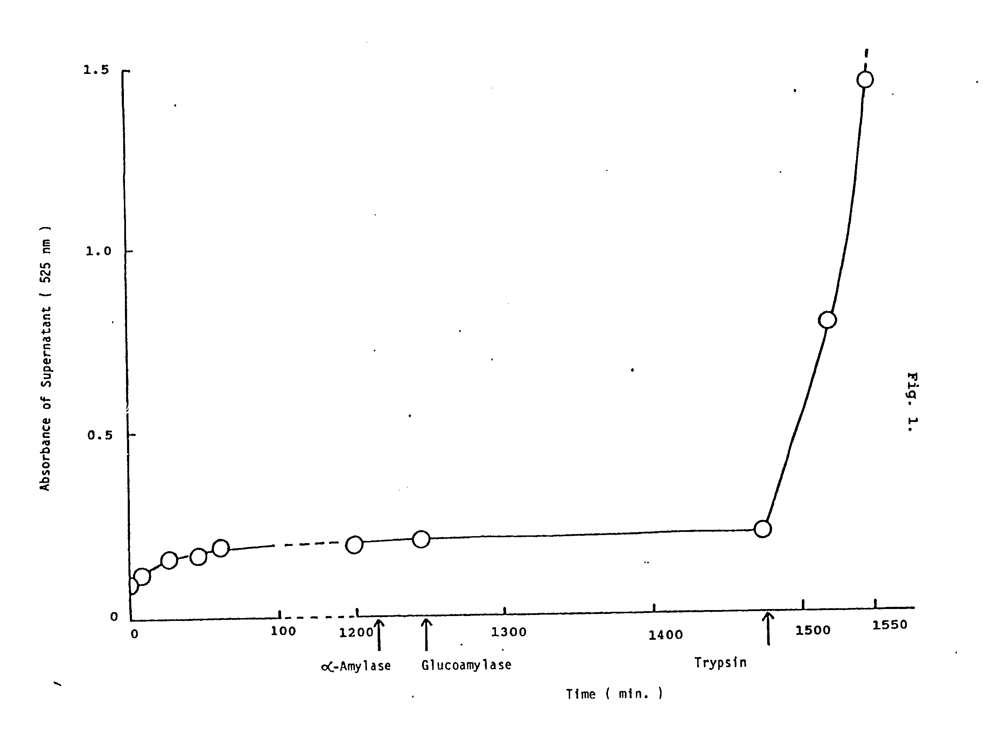

Alginate-casein-erythrosine beads were prepared as described in Example 1. After 1 hr the beads were filtered off and resuspended in water (20 cm³) kept at 37°C and stirred continuously. The absorbance of the supernatant was read at 525 nm at intervals. After approx 20.5 hr a solution containing bacterial α-amylase (1000 units in 200 μl) was added. At 21 hr a solution containing glucoamylase (200 μl, 97.6 μg) was added. At 25 hr, trypsin (177.6 mg in 200 μl water) was added. The absorbance at 525 nm was read after each addition. The result is shown in Figure 1. As can be seen from Figure 1, essentially no release of dye occurred following the addition of α-amylase or glucoamylase, since these enzymes are not proteases. However, addition of the protease trypsin caused immediate and rapid release of dye into the supernatant solution.

Example 3

Release of Casein-Bound EBV from Alginate Beads

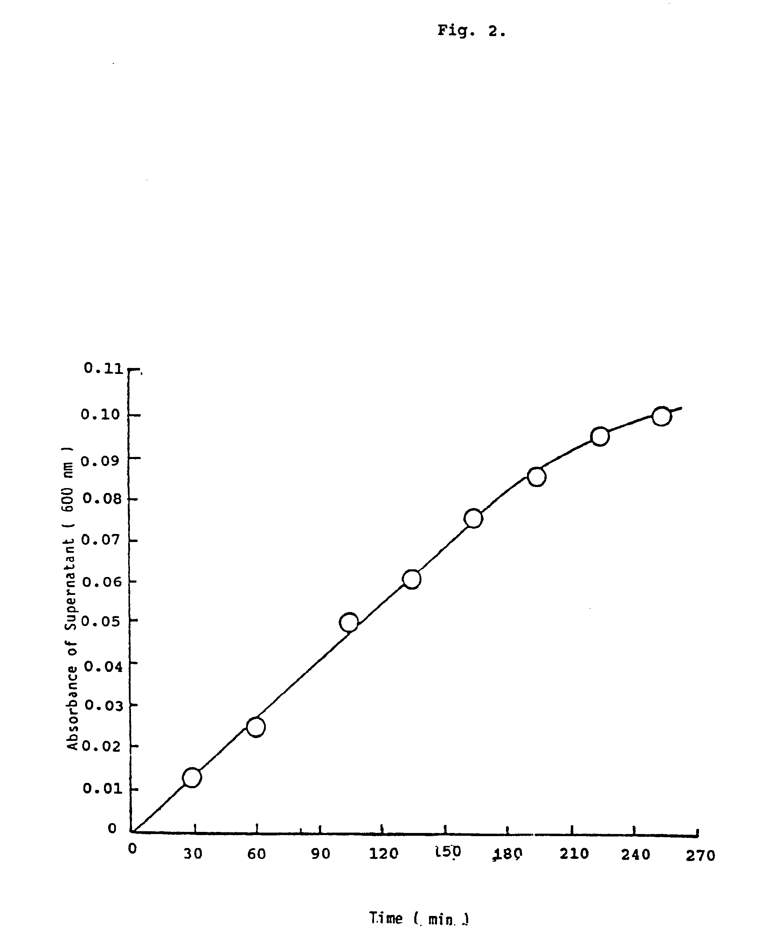

Using a peristaltic pump, 2.0 ml of a solution containing sodium alginate (1%), casein (1%) and EBV (7.7 μg, 0.2 mM) was dropped from a syringe needle into calcium chloride solution (20 cm³, 2%). The time required for pumping 2.0 ml was 3 min 20 sec. The beads were left in the CaCl₂ solution for 1-3 hr, then filtered off and rinsed with approx 50 ml of water. The batch of beads was then placed in a flask with buffer solution (10 cm³) containing the enzyme to be assayed. At intervals an aliquot of the supernatant was removed and, after its absorbance at 600 nm had been read, quickly replaced. Figure 2 shows the increase with time of absorbance of the supernatant solution when trypsin (18.6 µg in 0.02 M Tris buffer, pH 8.0) was used; it will be noted that the rate of dye release varies linearly with respect to time.

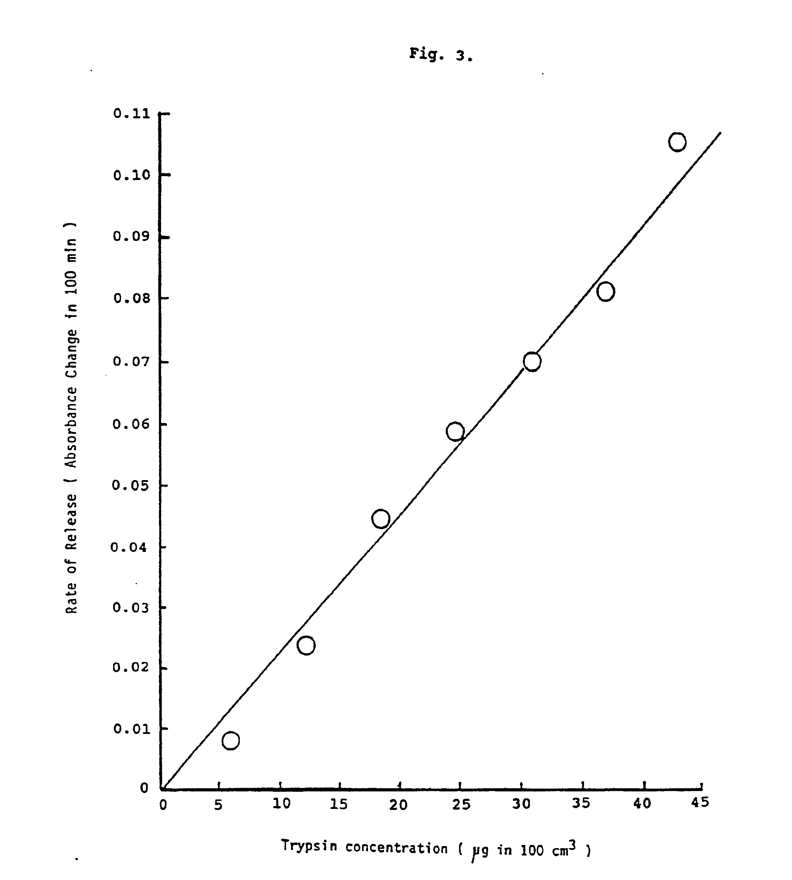

This experiment was repeated with varying concentrations of trypsin (6.2-43.4 µg in 10 cm³), and in each case the rate of increase in absorbance of the supernatant was obtained. Figure 3 shows that the rate of release of dye varied linearly with respect to enzyme concentration.

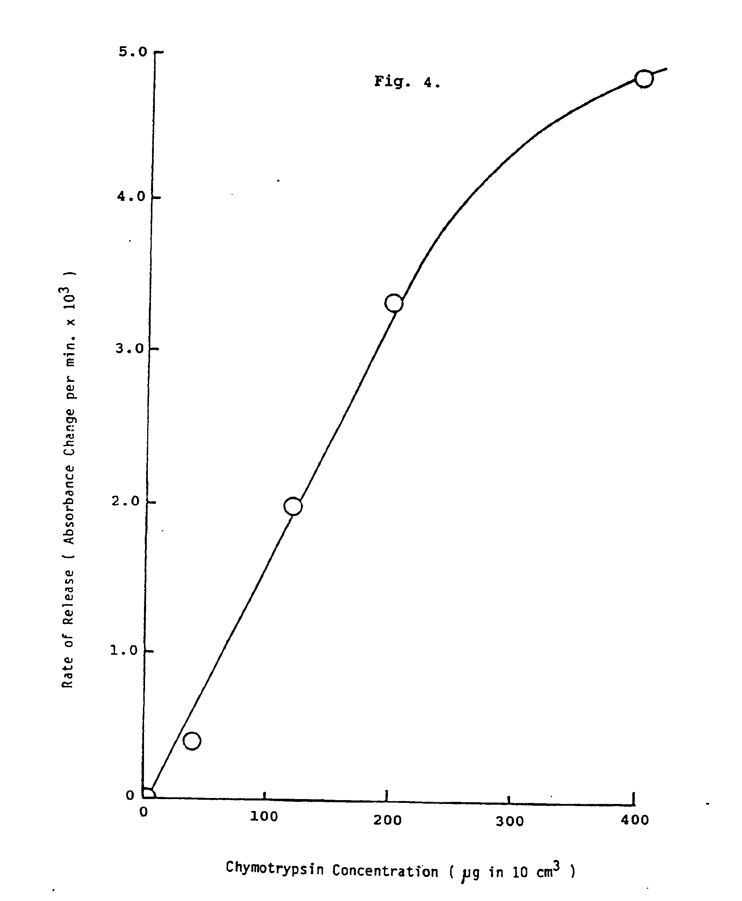

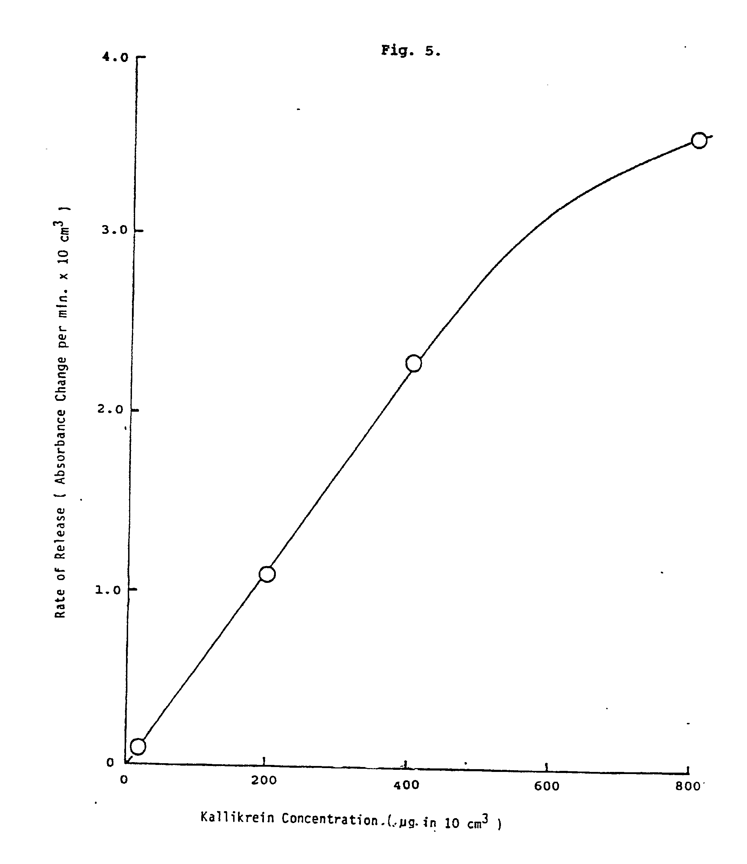

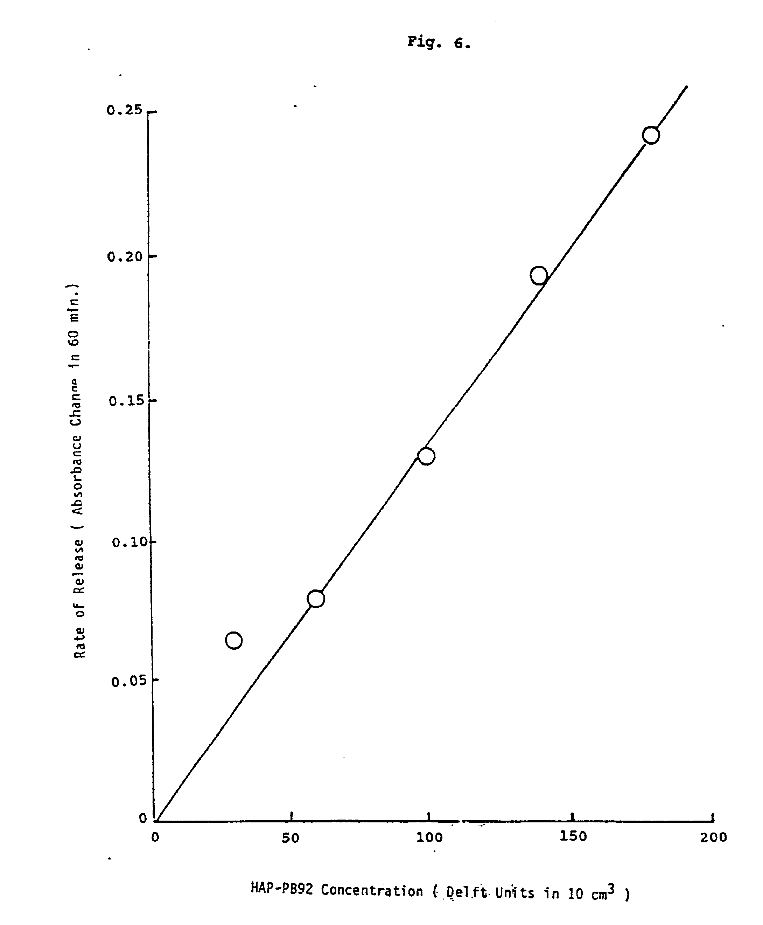

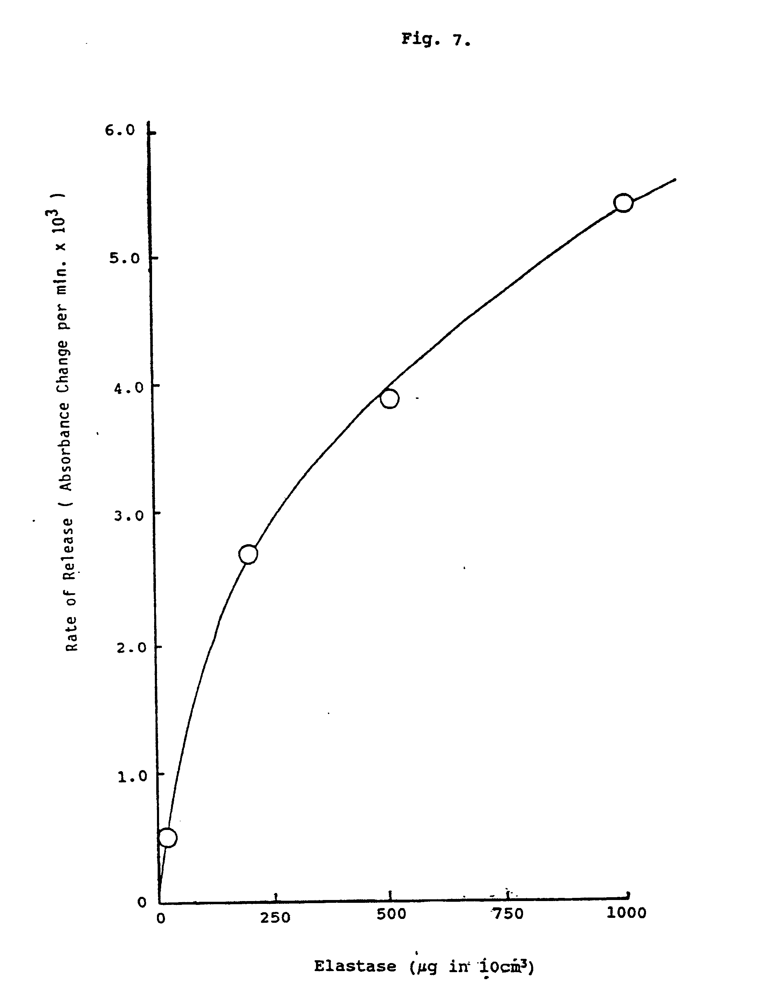

The experiment was also carried out using chymotrypsin (in 0.02 M Tris buffer, pH 8.0), kallikrein (in 0.02 M Tris buffer, pH 8.75), high alkaline proteinase HAP-PB92 (in 0.02 M Tris buffer, pH 8.5) and elastase (in 0.02 M Tris buffer, pH 8.75). Figures 4, 5, 6 and 7 respectively show the variation of dye release rate with enzyme concentration in each case. Each of these Figures shows a predictable, generally linear, variation of dye release rate with enzyme concentration.

## Example 4

### Release of Casein-Bound Tetracycline from Alginate Beads

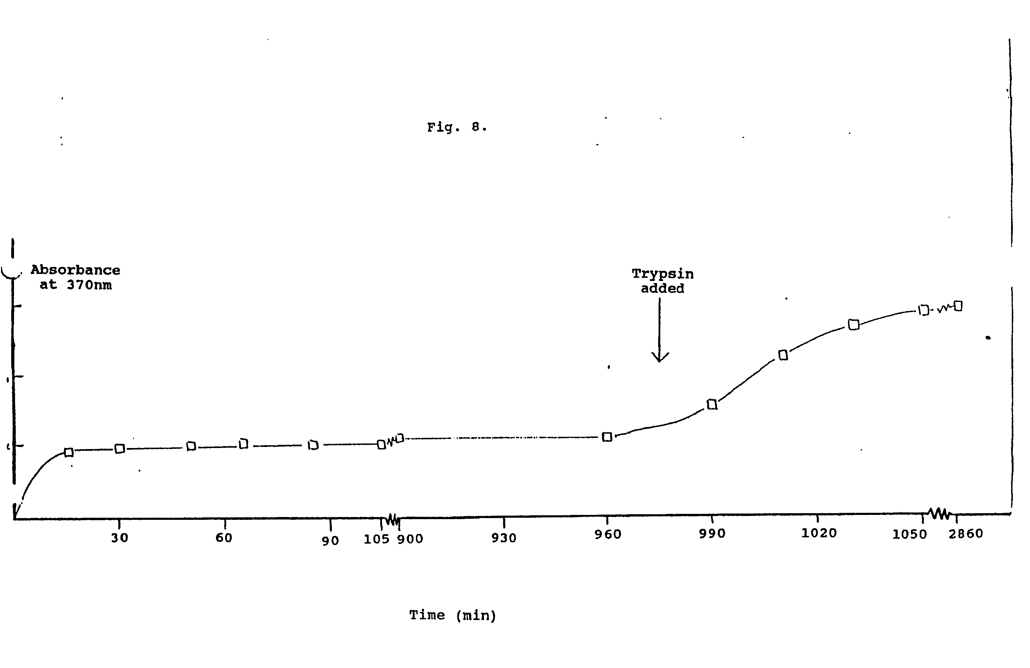

A set of tetracycline-casein-calcium alginate beads was prepared by dropping a solution (5 ml) of tetracycline (125 µg/ml), sodium alginate (1%) and denatured casein (1%) into calcium chloride solution (2%) at pH 11. After 4 hr the beads were transferred into 0.02 M Tris buffer pH 8.0 and the supernatant monitored at 370 nm for the release of tetracycline from the beads. Figure 8 shows that the release of tetracycline (26% of the total incorporated) was complete after 960 min. Then trypsin (142.5 µg in 200 µl) was added and the release of tetracycline was again monitored at 370 nm. Figure 8 shows the subsequent further release (up to 71% of the total incorporated) after a further 100 min.

## Claims

1. A controlled release formulation comprising:
   a gel matrix;
   a protein entrapped in the gel matrix; and
   an ingredient capable of binding to the entrapped protein,
   whereby, upon exposure of the formulation to a proteolytic enzyme-containing environment, the protein is degraded and the ingredient released from the protein and into the enzyme-containing environment.

2. A formulation of claim 1 wherein the ingredient is a drug.

3. A formulation of claim 2 wherein the drug is an antibiotic, hormone, hormone analog or chemotherapeutic agent, or mixture thereof.

4. A formulation of claim 1 wherein the protein is casein or serum albumin or other protein capable of being entrapped in the gel, or mixture thereof.

5. A formulation of claim 1 wherein the gel matrix is a matrix of a gel-forming polysaccharide or mixture of gel-forming polysaccharides selected from the group consisting of pectin, pectate, alginate, carageenin, xanthan gum guar gum, gum arabic, gum acacia, and carboxymethylcellulose.

6. A formulation of claim 5 wherein the gel matrix comprises a matrix of a salt of an alginate with calcium, magnesium, zinc, copper, manganese, aluminum or iron, or mixtures of calcium, magnesium, zinc, copper, manganese, aluminum or iron.

7. A formulation of claim 6 wherein the gel matrix comprises calcium alginate gel.

8. A formulation of claim 1 in the form of a bead, pellet, film, fiber or tube.

9. A formulation of claim 8 wherein the gel matrix comprises a calcium alginate gel bead.

10. A method for releasing the ingredient of claim 1 comprising adding one or more proteolytic enzymes to the controlled release formulation of claim 1.

11. A method of claim 10 wherein the proteolytic enzyme is trypsin.

12. A method of claim 10 wherein the ingredient is a drug which is released in a patient at a local site where the enzymes are active.

Fig. 1.

Fig. 2.

Time ( min. )

Fig. 3.

Fig. 4.

Fig. 5.

Fig. 6.

Fig. 7.

Rate of Release ( Absorbance Change per min. x 10³ )

Elastase (µg in 10cm³)

Fig. 8.

Absorbance
at 370nm

Trypsin
added

Time (min)

EP 0 447 100 A1

**European Patent Office**

## PARTIAL EUROPEAN SEARCH REPORT
which under Rule 45 of the European Patent Convention shall be considered, for the purposes of subsequent proceedings, as the European search report

Application number

EP 91301806.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP - A1 - 0 326 618 (NIPPON) <br> * Examples 29-31; page 5, line 22 - page 7, line 20; page 7, line 29 - page 8, line 17 * | 1-4,5, 8 | A 61 K 9/22 <br> A 61 K 9/26 <br> A 61 K 47/42 <br> A 61 K 47/48 |
| X | PATENT ABSTRACTS OF JAPAN, unexamined applications, C field, vol. 12, No. 181 THE PATENT OFFICE JAPANESE GOVERNMENT page 166 C 499 <br> * Kokai-No. 62-289 530 (EISAI CO LTD) * | 1-5,8, 10,11 | |
| X | EP - A1 - 0 095 968 (CENTRE) <br> * Claims 1,2,5,7,8; examples 2,3; page 1, lines 5-18 * | 1-5, 10,11 | |
| X | WO - A1 - 83/01 738 (SCHRÖDER U., MOSBACH K.) <br> * Abstract; example 12 * | 1,4,8 | **TECHNICAL FIELDS SEARCHED (Int. Cl.5)** <br> A 61 K 9/00 <br> A 61 K 47/00 |

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-9

Claims searched incompletely: 10,11

Claims not searched: 12

Reason for the limitation of the search:

Method for treatment of the human or animal body by therapy - EPC Article 52(4)

| Place of search | Date of completion of the search | Examiner |
|---|---|---|
| VIENNA | 14-06-1991 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.⁵) |
|---|---|---|---|
| | -- | | |
| X | US - A - 3 663 687 (EVANS R.L.) * Abstract; claims 1-5,8; column 5, lines 48-52 * | 1-5,8, 10,11 | |
| | -- | | |
| X | EP - A2 - 0 152 898 (MASSACHUSETTS) * Claims 1,2,4,5,10,11 * | 1-9 | |
| | -- | | |
| A | EP - A2 - 0 153 896 (LE PIVERT P.) * Claims 1,2; abstract * | 1,2 | |
| | -- | | |
| A | EP - A1 - 0 256 611 (SQUIBB) * Abstract; claims 1,6,7; page 12, line 15 - page 13, line 14 * | 1-9 | RECHERCHIERTE SACHGEBIETE (Int. Cl.⁵) |
| | ---- | | |